# EUROPEAN PATENT SPECIFICATION

(11) **EP 3 222 279 B1**
(45) Date of publication and mention of the grant of the patent: **17.11.2021**
(21) Application number: 16461511.4
(22) Date of filing: 21.03.2016
(51) Int. Cl.: A61K 31/47, A61K 31/495, A61K 9/20, A61K 9/28, A61P 11/06, A61P 11/02

(54) **ORAL PHARMACEUTICAL FORMULATION OF MONTELUKAST AND LEVOCETIRIZINE AND METHOD FOR ITS PRODUCTION**
ORALE PHARMAZEUTISCHE FORMULIERUNG VON MONTELUKAST UND LEVOCETIRIZIN UND VERFAHREN ZU DEREN HERSTELLUNG
PRÉPARATION PHARMACEUTIQUE ORALE DE MONTÉLUKAST ET DE LÉVOCÉTIRIZINE ET PROCÉDÉ POUR SA PRODUCTION

(43) Date of publication of application: 27.09.2017
(73) Proprietor: Invest Bielany Spolka z ograniczona odpowiedzialnoscia, 04-028 Warszawa (PL)
(72) Inventor: ZWOLINSKA-OBARSKA, Anna, 02-904 Warszawa (PL); LECHNIO, Joanna, 00-195 Warszawa Warszawa (PL); WISNIEWSKA, Anna, 01-873 Warszawa (PL); NICHTHAUSER, Joanna, 01-793 Warszawa (PL); POPIOLKIEWICZ, Joanna, 05-800 Pruszków (PL); URBANSKA, Agnieszka, 05-110 Jablonna (PL); OSINSKI, Andrzej, 00-875 Warszawa (PL); HEJDUK, Arkadiusz, 05-462 Warszawa (PL)
(74) Representative: BRANDPAT Patent and Trademark Attorneys Chlebicka Czyz Galazkiewicz Ziolkowski Professional Partnership

(56) References cited:
- BR-A2-102013 004 720
- RATHOD R T ET AL: "FDC of montelukast with levocetirizine: focus on bilayer technology", JOURNAL OF THE INDIAN MEDICAL ASSOCIATION, INDIAN MEDICAL ASSOCIATION, CALCUTTA, IN, vol. 107, no. 8, 1 August 2009 (2009-08-01), pages 562-564, XP008173253, ISSN: 0019-5847

## Description

### Technical Field

The subject of the invention is a tablet comprising levocetirizine and montelukast with good stability and a method for its production.

### Background Art

Levocetirizine ((*R*)-2-[[4-[(4-Chlorophenyl)phenylmethyl]-1-piperazi-nyl]ethoxy]acetic acid) which is the R-enantiomer of cetirizine is a piperazine derivative, potent and selective H1 receptor antagonist. Its structural formula is:

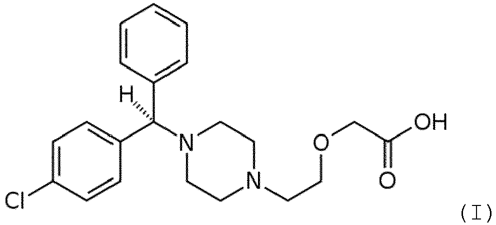

Levocetirizine is a third-generation antihistamine which binds to HI receptors with high affinity, even two times higher as compared to cetirizine. Levocetirizine has anti-allergic and anti-inflammatory activity. The studies conducted have shown that levocetirizine inhibits a comprehensive series of reactions that induce and disseminate allergic inflammation. Levocetirizine is commercially available in a form of dihydrochloride salt.

Montelukast is described chemically as 1-[[[(1R)-1-[3-[(1E)-2-(7-chloro-2-quinolinyl)ethenyl]phenyl]-3-[2-(1-hydroxy-1-methylethyl)phenyl]propyl]thio] methyl]cyclopropane acetic acid. Its structural formula is:

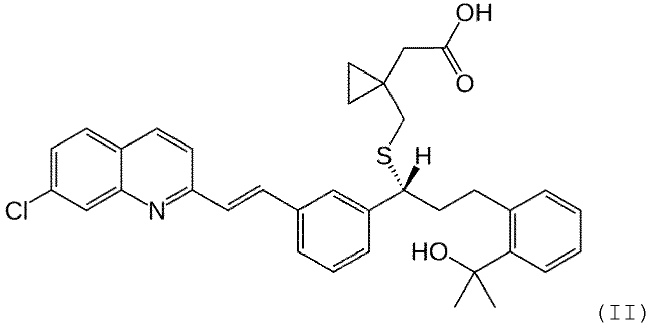

Montelukast is an orally-used, potent and selective antagonist of leukotriene D4 (LTD₄) which is effective on cysteinyl leukotriene receptor (CysLT1) in the respiratory tract. Montelukast is a powerful compound which treats asthma inflammation parameters significantly. Montelukast binds to CysLT1 receptor with a high affinity and selectivity and strongly inhibits physiological effects of LTC₄, LTD₄, LTE₄ in CysLT1 receptor without any agonist activity. Montelukast is commercially available in a form of sodium salt.

It is an object of the present invention to provide a tablet comprising a pharmaceutically acceptable salt of levocetirizine and pharmaceutically acceptable salt of montelukast as the active ingredients.

Combining montelukast with levocetirizine in one tablet gives additional benefits in comparison with one-component drugs and can be considered for patients whose quality of life is impaired by, for example, persistent allergic rhinitis or asthma.

This combination of montelukast and levocetirizine in a combination product facilitates dosing. A patient - instead of taking the two products served separately - is treated with one tablet, what significantly simplifies therapy compliance.

However, in forming the two active ingredients in one tablet, the problem of different acid-base characteristic of levocetirizine dihydrochloride and montelukast sodium arises: montelukast sodium is alkaline stable and levocetirizine dihydrochloride is acid stable. Therefore, when a matrix tablet is prepared, both drugs would be in contact and such a composition could be unstable during the shelf life of the formulation.

Levocetirizine dihydrochloride is freely soluble in acids and after tablet disintegration in stomach easily diffuses to surrounding medium. Montelukast sodium precipitates in contact with acids. Its precipitation rate is different in a case when montelukast sodium is mixed and compressed together with levocetirizine dihydrochloride, in comparison with a case when montelukast sodium is released from one-component tablet in the presence of levocetirizine dihydrochloride one-component tablet (similarly as in Singulair+Xyzal drug products).

The second problem is the lack of compatibility of both active ingredients (APIs): montelukast sodium and levocetirizine dihydrochloride. It is revealed by the increase of amount of impurities observed during compatibility studies.

The problem of mixing both active ingredients without losing their activity was examined by many researchers. One of the worked out solutions consists in separating both active ingredients in bilayer formulations.

Such formulations are disclosed in WO2012064305 wherein a bilayer tablet formulation is described, comprising montelukast and levocetirizine layers with at least two different diluents. Moreover, WO2013012199 (EP2731594) discloses a capsule formulation which comprises two separate layers, namely (1) a Montelukast layer and (2) a Levocetirizine layer. Publication WO2013055177 (EP2744485) discloses a hard capsule composite formulation comprising a capsule having one or more tablets placed in the capsule. Example 4 of the cited publication presents the preparation of a Montelukast containing coated tablet together with a Levocetirizine containing coated tablet charged in one capsule body.

WO2013103262 (EP2800558) discloses a pharmaceutical formulation for oral administration, which comprises two particle parts: (a) a first particle part comprising levocetirizine or a pharmaceutically acceptable salt thereof and an organic acid; and (b) a second particle part comprising montelukast or a pharmaceutically acceptable salt thereof.

WO2013154390 (EP2836207) describes a hard capsule composite formulation comprising two or more pharmaceutically active ingredients, wherein each pharmaceutically active ingredient is contained in a multi-unit spheroidal tablet (MUST) and a plurality of the MUSTs per each pharmaceutically active ingredient are encapsulated in the hard capsule. WO2014208915 discloses a complex granule formulation comprising:
(a) a first granular part comprising levocetirizine or a pharmaceutically acceptable salt thereof, cyclodextrin or a derivative thereof, and an alkalinizing agent; and
(b) a second granular part comprising montelukast or a pharmaceutically acceptable salt thereof, cyclodextrin or a derivative thereof, and an alkalinizing agent.

WO2015062466 discloses a granular composition which comprises montelukast sodium particles and levocetirizine hydrochloride particles; the montelukast particles comprise montelukast sodium, fillers, stabilizers, adhesion agent; the levocetirizine hydrochloride particles comprise levocetirizine hydrochloride, fillers, stabilizers, binders; the stabilizer is meglumine; the binder is hydroxypropyl-β-cyclodextrin.

BR102013004720 discloses a tablet comprising montelukast sodium and levocetirizine dihydrochloride, lactose monohydrate, microcrystalline cellulose, hydroxypropyl cellulose, croscarmellose sodium, magnesium stearate; the tablet being coated with a film, obtained in wet granulation manufacturing process comprising the stages of wet granulation of montelukast sodium with additives, blend the granules with levocetirizine dihydrochloride and additives, and press the product to create tablets; optionally covered with coating and put in capsules.

According to R.T. Rathord, J Indian Med Assoc. 2009 Aug; 107(8):562-4, an attempt to prepare a matrix tablet comprising montelukast sodium and levocetirizine dihydrochloride was unsuccessful, due to poor stability of the prepared formulation during shelf life. Even in a case of bilayer tablet, complete physical separation of the active ingredients: montelukast sodium and levocetirizine dihydrochloride is not possible and one may observe an increase in impurities originating from montelukast sodium.

BR 102013004720 describes an oral pharmaceutical composition comprising montelukast and levocetirizine obtained by a process comprising mixing montelukast with different excipients, granulating this mixture and mixing it with levocetirizine.

The methods described above, moreover, require the use of modern, very expensive equipment and - what is more - such manufacturing processes are difficult to validate.

The aim of this invention is to provide a stable montelukast and levocetirizine composition which does not require complicated and expensive equipment.

### Disclosure of the Invention

The present invention provides a pharmaceutical formulation for oral administration in a form of a film coated tablet which is obtainable by direct compression of the tablet core for treating asthma and accompanying allergic rhinitis which comprises
(a) levocetirizine or a pharmaceutically acceptable salt thereof mixed together with
(b) montelukast or a pharmaceutically acceptable salt thereof,
wherein levocetirizine or its salt and montelukast or its salt are placed together in a tablet core giving no significant change in storage stability wherein the content of montelukast impurity C of formula (III) 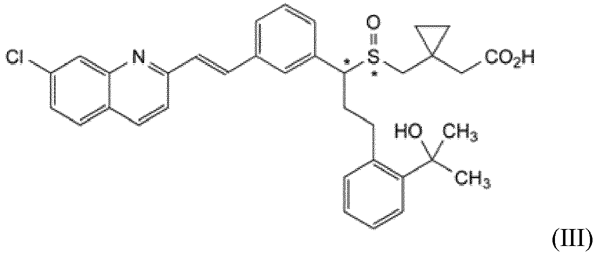 is not higher than 0.5% based on nominal montelukast content when the tablet is stored at 40°C±2°C and 75%±5% relative humidity for 6 months.

Storage stability, in accordance with ICH guideline CPMP/QWP/122/02 is defined as storage at long term conditions (25°C ± 2°C/60% RH ± 5% RH during 12 months) or accelerated conditions (40°C ± 2°C/75% RH ± 5% RH during 6 months) without "significant change" in drug product specification. "Significant change" for a drug product is defined as:
1. A 5% change in assay from its initial value;
2. Any degradation product's exceeding its acceptance criterion;
3. Failure to meet the acceptance criteria for appearance, physical attributes, and functionality test, however, some changes in physical attributes (e.g., softening of suppositories, melting of creams) may be expected under accelerated conditions;
4. Failure to meet the acceptance criteria for dissolution.

In accordance with the present invention the pharmaceutical formulation has a form of the tablet prepared by direct compression.

The film-coated tablet is a solid single-dose preparation consisting of the tablet coated with a thin polymeric film that dissolves within a few minutes in the gastrointestinal fluid. The film-coated tablets for oral use are intended to release active substances at a rate which is not significantly delayed compared to that of the uncoated tablet, i.e. within a few minutes.

All percentage (%) values presented in the present invention refer to percentage by weight (% w/w)
The pharmaceutically acceptable salt of levocetirizine is preferably levocetirizine dihydrochloride and the pharmaceutically acceptable salt of montelukast is preferably montelukast sodium.

The pharmaceutical formulation according to the invention comprises

| | |
|---|---|
| Levocetirizine dihydrochloride | in a pharmaceutically effective amount, |
| Montelukast sodium | in a pharmaceutically effective amount, |
| Lactose monohydrate | in the amount of 10 - 90%, |
| Microcrystalline cellulose | in the amount of 1 - 76%, |
| Hydroxypropylcellulose | in the amount of 0.5 - 4%, |
| Croscarmellose sodium | in the amount of 0.5 - 4%, |
| Magnesium stearate | in the amount of 0.5 - 1% of the total mass of the tablet core. |

In preferred embodiments the pharmaceutical formulation comprises

| | |
|---|---|
| Levocetirizine dihydrochloride | in a pharmaceutically effective amount, |
| Montelukast sodium | in a pharmaceutically effective amount, |
| Lactose monohydrate | in the amount of 10% or 15% or 20% or 25% or 30% or 35% or 40% or 45% or 50% or 55% or 60% or 65% or 70% or 75% or 80% or 85% or 90%, |
| Microcrystalline cellulose | in the amount of 1% or 5% or 10% or 15% or 20% or 25% or 30% 35% or 40% or 45% or 50% or 55% or 60% or 65% or 70% or 75% or 76%, |
| Hydroxypropylcellulose | in the amount of 0.5% or 1% or 1.5% or 2% or 2.5% or 3% or 3.5% or 4%, |
| Croscarmellose sodium | in the amount of 0.5% or 1% or 1.5% or 2% or 2.5% or 3% or 3.5% or 4%, |
| Magnesium stearate | in the amount of 0.5% or 0.6% or 0.7% or 0.8% or 0.9% or 1% of the total mass of the tablet core. |

The pharmaceutical formulation preferably comprises levocetirizine dihydrochloride in the amount of 5 mg and montelukast sodium in the amount equivalent to 10 mg of montelukast free acid.

The pharmaceutical formulation preferably gives no significant change in storage stability after 6 months in accelerated conditions (40°C±2°C, 75%±5% RH), where the significant change is 5% change in assay of montelukast sodium from its initial value.

It is another object of the present invention to provide a method for preparing the pharmaceutical formulation for oral administration for use in treating asthma and accompanying allergic rhinitis which comprises levocetirizine or a pharmaceutically acceptable salt thereof and montelukast or a pharmaceutically acceptable salt thereof, whereby the method comprises the steps of:
(I) mixing levocetirizine or the pharmaceutically acceptable salt thereof and at least one pharmaceutically acceptable additive,
(II) mixing montelukast or the pharmaceutically acceptable salt thereof and at least one pharmaceutically acceptable additive,
(III) blending the mixture obtained in stage (I) with the mixture obtained in stage (II) and at least one pharmaceutically acceptable additive,
(IV) tableting the mixture obtained in stage (III), and
(V) film-coating the tablets.

The tablets can further be packed into blisters.

Preferably acceptable additive is selected from a group comprising: microcrystalline cellulose, powdered cellulose, silicified microcrystalline cellulose, ethers of cellulose including hydroxypropylcellulose and hydroxypropylmethylcellulose, lactose, lactose monohydrate, lactose anhydrous, spray dried lactose, croscarmellose or its salt.

It is preferred that the pharmaceutically acceptable salt of levocetirizine is levocetirizine dihydrochloride and the pharmaceutically acceptable salt of montelukast is montelukast sodium.

Preferred blisters are made of material or laminate, which ensures high protection against humidity, oxygen and UV radiation. It is preferred that blisters are made of PVC, OPA (oriented polyamide), aluminium foil, PCTFE (e.g. Aclar), PVDC (polyvinylidene chloride), PVDC-coated PVC, PVC/PE/PCTFE laminate, CFF (Cold-Form Foil), COC (Cyclic Olefin Copolymer) or combination thereof.

### Brief Description of Drawings

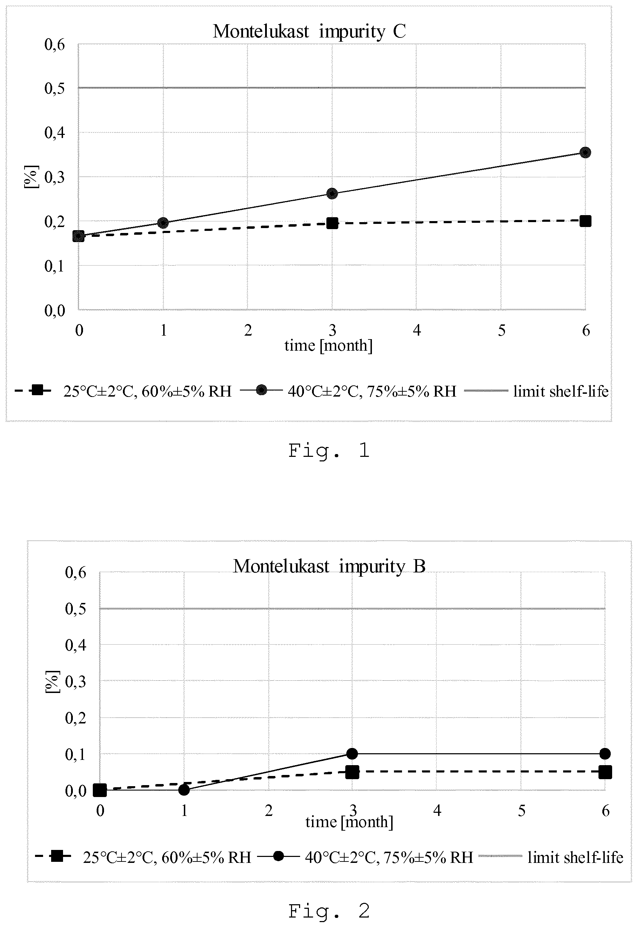

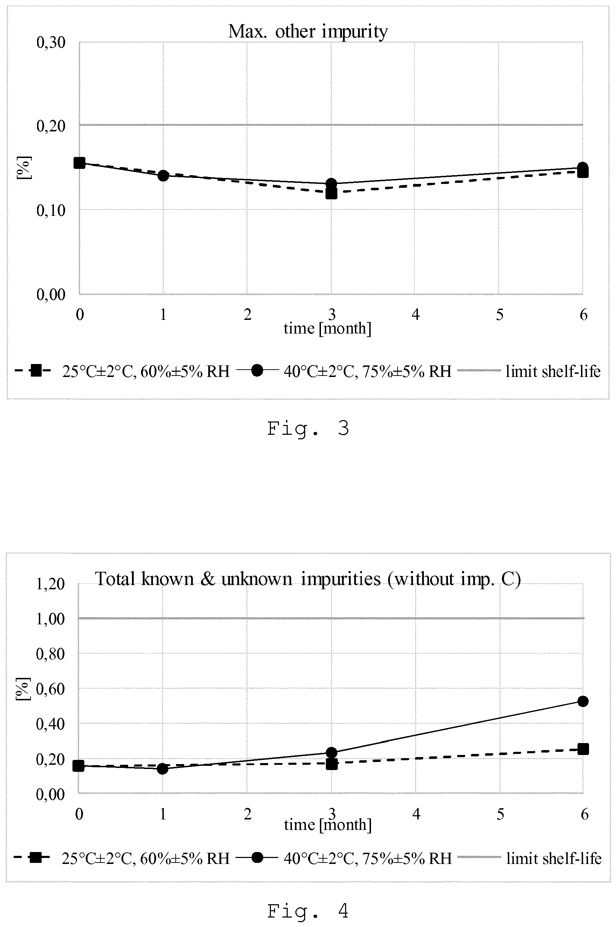

The invention and its effectiveness are presented on the attached drawings which present the results of impurities test and shelf-life specification limits for specified impurities in two stability conditions for a tested product in a form of film-coated tablets, prepared according to Example 5. The results (Figures 1-4) represent mean value of two batches of the film-coated tablets packed into blisters and put into climatic chambers.
Fig. 1 Montelukast impurity C level in stability studies of the film-coated tablets;
Fig. 2 Montelukast impurity B level in stability studies of the film-coated tablets;
Fig. 3 Maximum other impurity level in stability studies of the film-coated tablets;
Fig. 4 Total known and unknown impurities (without impurity C) in stability studies of the film-coated tablets.

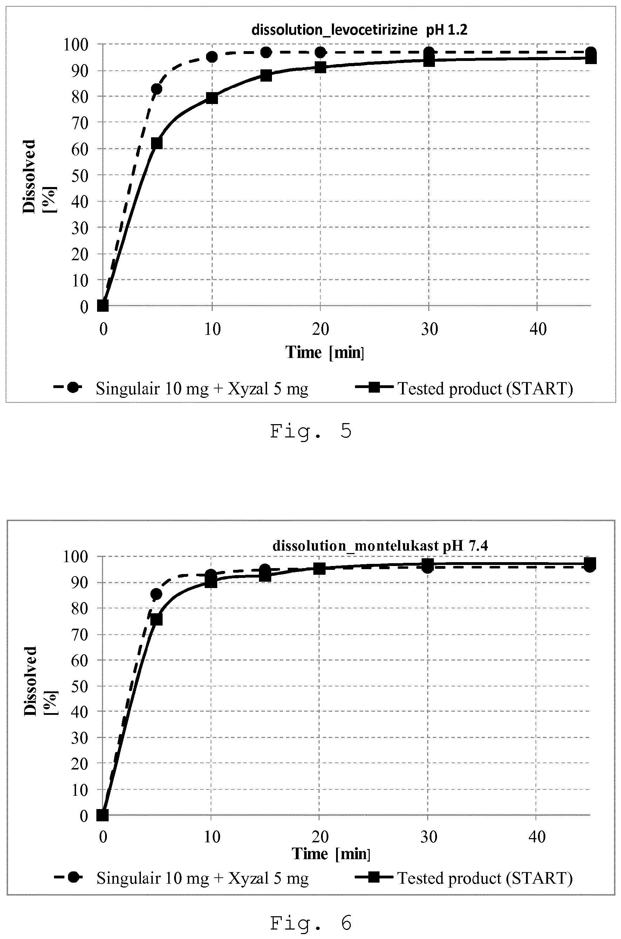

In vitro dissolution tests for a reference product and the tested product according to Example 5 of the present invention are presented on Fig. 5-6 and in Tables 1 and 2.

Fig. 5 Comparison of levocetirizine dihydrochloride dissolution profiles for the product according to the present invention and a reference product: Xyzal (5 mg of levocetirizine dihydrochloride) with Singulair (10 mg of montelukast) in pH 1.2;

Fig. 6. Comparison of montelukast dissolution profiles for the product according to the present invention and the reference product: Singulair (10 mg of montelukast) with Xyzal (5 mg of levocetirizine dihydrochloride) in pH 7.4 .

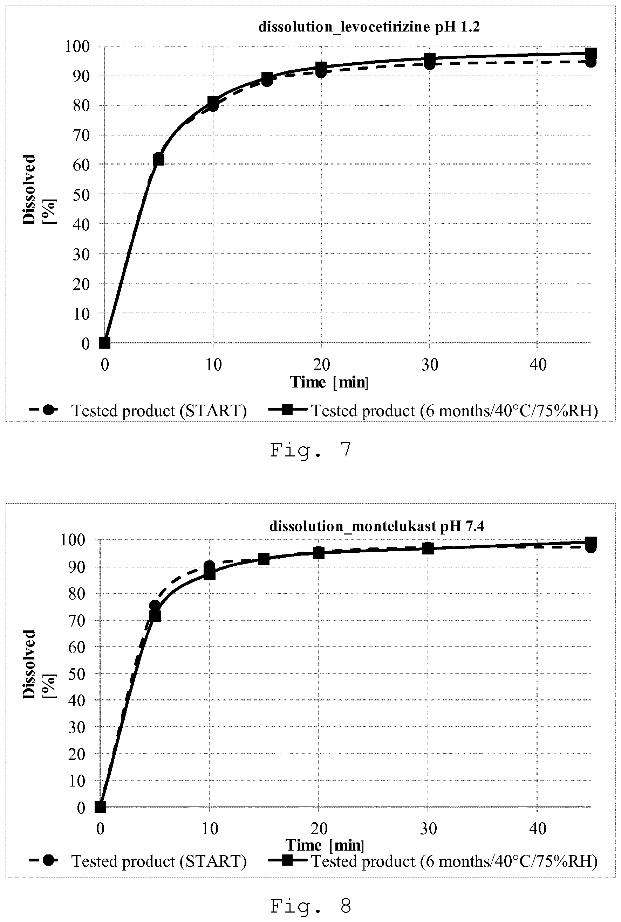

Comparison of dissolution profiles for the product according to the present invention at the beginning of stability studies and after 6 months in accelerated conditions (40°C±2°C, 75%±5% RH) is presented on Figs. 7-8 and in Tables 3 and 4.

Fig. 7 Comparison of levocetirizine dissolution profiles in pH 1.2 for the product according to the present invention at the beginning of stability studies and after 6 months in accelerated conditions (40°C±2°C, 75%±5% RH);

Fig. 8 Comparison of montelukast dissolution profiles in pH 7.4 for the product according to the present invention at the beginning of stability studies and after 6 months in accelerated conditions (40°C±2°C, 75%±5% RH)..

### The Best Mode for Carrying out the Invention

Herein we present Examples which are intended to further illustrate the present invention.

### Example 1

### Procedure of the preparation of the film-coated tablet formulation

All batches were manufactured according to the following steps:
All ingredients were weighed in appropriate amounts and sieved.

Levocetirizine dihydrochloride, croscarmellose sodium and optionally citric acid (see Example 4) and half of lactose monohydrate were manually mixed and then preliminary blended for 10 minutes.

Montelukast sodium and a half of microcrystalline cellulose were manually mixed and then preliminary blended for 10 minutes.

Both obtained mixtures were sieved.

Then the blender was loaded with remaining amount of lactose monohydrate, followed by both mixtures, hydroxypropylcellulose and remaining amount of microcrystalline cellulose. The blending operation was continued for 15 minutes.

Obtained product mixture was sieved.

After screening, the blending operation was continued for 15 minutes to obtain a homogenous mixture.

Finally the blender was loaded with magnesium stearate and the blending operation continued for 2 minutes.

The resulting blend was tableted using a rotary, production scale tablet press.

The resulting tablet cores were coated with 15% aqueous solution of Opadry to achieve a weight gain of approx. 3%. Opadry is a complete film coating system, based on hypromellose.

Film-coated tablets are packed into blisters containing OPA/Alu/PVC and thin, hard aluminum foil.

All film-coated tablets described in the following examples were prepared in accordance with Example 1.

### Example 2

The film-coated tablet was prepared according to the procedure described in Example 1. Tablet core nominal weight was 1200 mg.

| **Raw material** | **Quantity [mg/tablet]** | **Quantity [%]** |
|---|---|---|
| Levocetirizine dihydrochloride | 5.00 | 0.42% |
| Montelukast sodium | 10.40 | 0.86% |
| Lactose monohydrate | 707.00 | 58.92% |
| Microcrystalline cellulose | 396.00 | 33.00% |
| Hydroxypropylcellulose | 36.00 | 3.00% |
| Croscarmellose sodium | 36.00 | 3.00% |
| Magnesium stearate | 9.60 | 0.80% |
| **Nominal core tablet weight** | **1200.00** | 100.00% |

### Example 3

The film-coated tablet was prepared according to procedure described in Example 1. Tablet core nominal weight was 200 mg.

| **Raw material** | **Quantity [mg/tablet]** | **Quantity [%]** |
|---|---|---|
| Levocetirizine dihydrochloride | 5.00 | 2.50% |
| Montelukast sodium | 10.40 | 5.20% |
| Lactose monohydrate | 105.00 | 52.50% |
| Microcrystalline cellulose | 66.00 | 33.00% |
| Hydroxypropylcellulose | 6.00 | 3.00% |
| Croscarmellose sodium | 6.00 | 3.00% |
| Magnesium stearate | 1.60 | 0.80% |
| **Nominal core tablet weight** | **200.00** | 100.00% |

### Example 4

The film-coated tablet was prepared according to procedure described in Example 1. Tablet core nominal weight was 200 mg.

| **Raw material** | **Quantity [mg/tablet]** | **Quantity [%]** |
|---|---|---|
| Levocetirizine hydrochloride | 5.00 | 2.50% |
| Montelukast sodium | 10.40 | 5.20% |
| Lactose monohydrate | 100.00 | 50.00% |
| Microcrystalline cellulose | 61.00 | 30.50% |
| Citric acid | 10.00 | 5.00% |
| Hydroxypropylcellulose | 6.00 | 3.00% |
| Croscarmellose sodium | 6.00 | 3.00% |
| Magnesium stearate | 1.60 | 0.80% |
| **Nominal core tablet weight** | 200.00 | 100.00% |

### Example 5

The film-coated tablet was prepared according to procedure described in Example 1. Tablet core nominal weight was 200 mg.

| **Raw material** | **Quantity [mg/tablet]** | **Quantity [%]** |
|---|---|---|
| Levocetirizine dihydrochloride | 5.00 | 2.50% |
| Montelukast sodium | 10.40 | 5.20% |
| Lactose monohydrate | 138.00 | 69.00% |
| Microcrystalline cellulose | 33.00 | 16.50% |
| Hydroxypropylcellulose | 6.00 | 3.00% |
| Croscarmellose sodium | 6.00 | 3.00% |
| Magnesium stearate | 1.60 | 0.80% |
| Nominal core tablet weight | 200.00 | 100.00% |

### Example 6

The film-coated tablet was prepared according to procedure described in Example 1. Tablet core nominal weight was 200 mg.

| **Raw material** | **Quantity [mg/tablet]** | **Quantity [%]** |
|---|---|---|
| Levocetirizine dihydrochloride | 5.00 | 2.50% |
| Montelukast sodium | 10.40 | 5.20% |
| Lactose monohydrate | 121.50 | 60.75% |
| Microcrystalline cellulose | 49.50 | 24.75% |
| Hydroxypropylcellulose | 6.00 | 3.00% |
| Croscarmellose sodium | 6.00 | 3.00% |
| Magnesium stearate | 1.60 | 0.80% |
| **Nominal core tablet weight** | **200.00** | 100.00% |

### Example 7

The film-coated tablet was prepared according to procedure described in Example 1. Tablet core nominal weight was 200 mg.

| **Raw material** | **Quantity [mg/tablet]** | **Quantity [%]** |
|---|---|---|
| Levocetirizine dihydrochloride | 5.00 | 2.50% |
| Montelukast sodium | 10.40 | 5.20% |
| Lactose monohydrate | 121.50 | 60.75% |
| Microcrystalline cellulose | 49.50 | 24.75% |
| Hydroxypropylcellulose | 8.00 | 4.00% |
| Croscarmellose sodium | 4.00 | 2.00% |
| Magnesium stearate | 1.60 | 0.80% |
| **Nominal core tablet weight** | 200.00 | 100.00% |

### Example 8

The film-coated tablet was prepared according to procedure described in Example 1. Tablet core nominal weight was 200 mg.

| **Raw material** | **Quantity [mg/tablet]** | **Quantity [%]** |
|---|---|---|
| Levocetirizine dihydrochloride | 5.00 | 2.50% |
| Montelukast sodium | 10.40 | 5.20% |
| Lactose monohydrate | 179.60 | 89.80% |
| Microcrystalline cellulose | 2.00 | 1.00% |
| Hydroxypropylcellulose | 1.00 | 0.50% |
| Croscarmellose sodium | 1.00 | 0.50% |
| Magnesium stearate | 1.00 | 0.50% |
| **Nominal core tablet weight** | 200.00 | 100.00% |

### Example 9

The film-coated tablet was prepared according to procedure described in Example 1. Tablet core nominal weight was 200 mg.

| **Raw material** | **Quantity [mg/tablet]** | **Quantity [%]** |
|---|---|---|
| Levocetirizine dihydrochloride | 5.00 | 2.50% |
| Montelukast sodium | 10.40 | 5.20% |
| Lactose monohydrate | 19.60 | 9.80% |
| Microcrystalline cellulose | 151.40 | 75.70% |
| Hydroxypropylcellulose | 6.00 | 3.00% |
| Croscarmellose sodium | 6.00 | 3.00% |
| Magnesium stearate | 1.60 | 0.80% |
| **Nominal core tablet weight** | 200.00 | 100.00% |

### Example 10

### Stability studies

Despite the interaction of both active substances levocetirizine dihydrochloride and montelukast sodium, the stable formulation of the invention containing both APIs in one pharmaceutical form - film-coated tablets, was achieved and positive results of stability studies for all specified parameters were received, as demonstrated on the drawings reflecting the following tests.

Two pilot batches prepared according to Example 5 were tested in stability studies according to ICH guideline CPMP/QWP/122/02 in two types of stability conditions:
1. Long term conditions: temperature 25°C±2°C, relative humidity 60%±5% (RH)
2. Accelerated conditions: temperature 40°C±2°C, relative humidity 75%±5% (RH).

For analysis of impurities validated UPLC method was used.

Main known degradation products of the final tablet product are: impurity B being montelukast impurity and impurity C being also montelukast impurity:

| | |
|---|---|
| Montelukast Impurity B: | [1-[[[(1*R*)-1-[3-[(*E*)-2-(7-chloroquinolin-2-yl)ethenyl]phenyl]-3-[2-(1-methylethenyl)phenyl]propyl]sulfanyl]methyl]cyclopropyl] acetic acid |
| Montelukast Impurity C | [1-[[[1-[3-[(*E*)-2-(7-chloroquinolin-2-yl)ethenyl]phenyl]-3-[2-(1-hydroxy-1-methylethyl)phenyl]propyl]sulfinyl]methyl] cyclopropyl] acetic acid |
| | 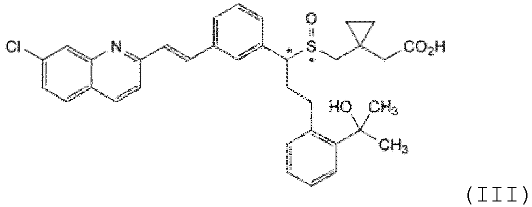 |

Montelukast Impurity C exists as two diastereoisomers due to a chiral nature of the sulfoxide moiety. The level of montelukast impurity C is determined as a sum of the levels for both diastereoisomers. Results for impurities test and shelf-life specification limits for specified impurities in two stability studies conditions are presented on Figures 1-4 for two batches of the tested product from Example 5. It can be seen that "significant change" does not occur after 6 months in accelerated conditions (40°C ± 2°C/75% RH ± 5% RH).

The results for tested product after 6 months in accelerated conditions (40°C±2°C, 75%±5% RH) are presented in the table below:

| Impurity | Results after 6 months in accelerated conditions (40°C±2°C, 75%±5% RH) |
|---|---|
| Max. known impurity (montelukast impurity C) | <0.4% |
| Max. other impurity (unspecified impurity) | <0.2% |
| Total known and unknown impurities (without impurity C) | <0.6% |

The level of impurities refers to the amount of the active ingredient.

Considering the fact, that levocetirizine dihydrochloride and montelukast have different solubility, two different dissolution media were chosen.

Selection of apparatus, dissolution volume and rotation speed for each of the compounds of drug product was performed based on characterization of reference drug products Singulair 10 mg film-coated tablets and Xyzal 5mg film-coated tablets.

Reference drug product with both active pharmaceutical ingredients combination (montelukast sodium with levocetirizine dihydrochloride) is not available on the European market. Therefore two separate reference products were analyzed together in one system. This approach allowed to estimate interactions of both substances.

For analysis of dissolution profiles validated UPLC method was used.

*In vitro* dissolution test for the reference and tested products are presented in Tables 1 and 2 and on Figures 5 and 6. The tested product is the film-coated tablet prepared according to Example 5. Percent dissolution values are the mean values from 12 individual dissolution tests.

**Table 1. The summary of results (12 units) of levocetirizine dihydrochloride dissolution profiles for tested product and Xyzal 5 mg with Singulair 10 mg in 0.1 M HCl pH 1.2.**

| **Product name** | **Dissolution** | **Time points [min]** | | | | | |
|---|---|---|---|---|---|---|---|
| | | **5** | **10** | **15** | **20** | **30** | **45** |
| Xyzal 5 mg with Singulair 10 mg | [%] | 82.9 | 95.2 | 96.9 | 96.9 | 97.0 | 97.0 |
| Tested product | [%] | 62.1 | 79.5 | 88.2 | 91.2 | 93.8 | 94.7 |

**Table 2. The summary of results (12 units) of montelukast dissolution profiles for tested product and Singulair 10 mg with Xyzal 5 mg in pH 7.4.**

| **Product name** | **Dissolution** | **Time points [min]** | | | | | |
|---|---|---|---|---|---|---|---|
| | | **5** | **10** | **15** | **20** | **30** | **45** |
| Singulair 10 mg with Xyzal 5 mg | [%] | 85.3 | 93.0 | 94.8 | 95.2 | 95.7 | 95.8 |
| Tested product | [%] | 75.5 | 90.2 | 92.7 | 95.4 | 97.1 | 97.1 |

According to Appendix I of the "Guideline on the Investigation of Bioequivalence" (CPMP/EWP/QWP/1401/98) where more than 85% of the drug is dissolved within 15 minutes, dissolution profiles may be accepted as similar without further mathematical evaluation; a value between 50 and 100 suggests that the two dissolution profiles are similar. For montelukast sodium, in pH 7.4 the amount of montelukast dissolved from the tested product from Example 5 and from Singulair 10 mg film-coated tablets in the presence of Xyzal 5 mg film-coated tablets, after 15 minutes of dissolution test, is greater than 85%.

For levocetirizine dihydrochloride, in 0.1 M HCl pH 1.2 the amount of Levocetirizine dihydrochloride dissolved from tested product from Example 5 and from Singulair 10 mg film-coated tablets in the presence of Xyzal 5 mg film-coated tablets, after 15 minutes of dissolution test, is greater than 85%.

The presented results of dissolution profiles of pilot batches of the tablets according to the invention allow the conclusion that the reference drug products and tested product can be assumed similar.

Dissolution profiles of the tested product are stable during stability studies.

Comparison of dissolution profiles for the tested product from Example 5 at the beginning of stability studies and after 6 months in accelerated conditions (40°C±2°C, 75%±5% RH) are presented in Tables 3 and 4 and on Figures 7 and 8.

**Table 3. The summary of results (12 units) of levocetirizine dihydrochloride dissolution profiles for the tested product at the beginning of stability studies and after 6 months in accelerated conditions (40°C±2°C, 75%±5% RH) in 0.1 M HCl pH 1.2.**

| **Product name** | **Dissolution** | **Time points [min]** | | | | | |
|---|---|---|---|---|---|---|---|
| | | **5** | **10** | **15** | **20** | **30** | **45** |
| Tested product beginning of stability studies | [%] | 62.1 | 79.5 | 88.2 | 91.2 | 93.8 | 94.7 |
| Tested product 6 months/ 40°C±2°C, 75%±5% RH | [%] | 61.5 | 81.0 | 89.2 | 92.8 | 95.8 | 97.5 |

**Table 4. The summary of results (12 units) of montelukast dissolution profiles for the tested product at the beginning of stability studies and after 6 months in accelerated conditions (40°C±2°C, 75%±5% RH) in pH 7.4.**

| **Product name** | **Dissolution** | **Time points [min]** | | | | | |
|---|---|---|---|---|---|---|---|
| | | **5** | **10** | **15** | **20** | **30** | **45** |
| Tested product beginning of stability studies | [%] | 75.5 | 90.2 | 92.7 | 95.4 | 97.1 | 97.1 |
| Tested product 6 months/ 40°C±2°C, 75%±5% RH | [%] | 71.5 | 87.4 | 92.8 | 95.0 | 96.6 | 99.1 |

In the consequence of the above results, the positive effect of bioequivalence studies is received and the stability of the formulation obtained is confirmed.

## Claims

1. A pharmaceutical formulation for oral administration in a form of a film coated tablet which is obtainable by direct compression of the tablet core and which comprises
(a) levocetirizine or a pharmaceutically acceptable salt thereof, mixed together with
(b) montelukast or a pharmaceutically acceptable salt thereof,
where (a) and (b) are placed together in the tablet core
and wherein the content of montelukast impurity C of formula (III) 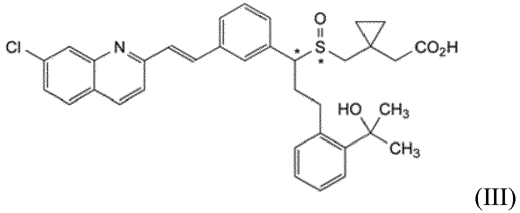 is not higher than 0.5% based on nominal montelukast content when the tablet is stored at 40°C±2°C and 75%±5% relative humidity for 6 months.

2. The pharmaceutical formulation according to claim 1, wherein the pharmaceutically acceptable salt of levocetirizine is levocetirizine dihydrochloride and the pharmaceutically acceptable salt of montelukast is montelukast sodium.

3. The pharmaceutical formulation according to any preceding claim comprising
| | |
|---|---|
| Levocetirizine dihydrochloride | in a pharmaceutically effective amount, |
| Montelukast sodium | in a pharmaceutically effective amount, |
| Lactose monohydrate | in the amount of 10% - 90%, |
| Microcrystalline cellulose | in the amount of 1% - 76%, |
| Hydroxypropylcellulose | in the amount of 0.5% - 4%, |
| Croscarmellose sodium | in the amount of 0.5% - 4%, |
| Magnesium stearate | in the amount of 0.5% - 1% |
or the total mass or the tablet core.

4. The pharmaceutical formulation according to any preceding claim comprising
| | |
|---|---|
| Levocetirizine dihydrochloride | in a pharmaceutically effective amount, |
| Montelukast sodium | in a pharmaceutically effective amount, |
| Lactose monohydrate | in the amount of 10% or 15% or 20% or 25% or 30% or 35% or 40% or 45% or 50% or 55% or |
| | 60% or 65% or 70% or 75% or 80% or 85% or 90%, |
| Microcrystalline cellulose | in the amount of 1% or 5% or 10% or 15% or 20% or 25% or 30% 35% or 40% or 45% or 50% or 55% or 60% or 65% or 70% or 75% or 76%, |
| Hydroxypropylcellulose | in the amount of 0.5% or 1% or 1.5% or 2% or 2.5% or 3% or 3.5% or 4%, |
| Croscarmellose sodium | in the amount of 0.5% or 1% or 1.5% or 2% or 2.5% or 3% or 3.5% or 4%, |
| Magnesium stearate | in the amount of 0.5% or 0.6% or 0.7% or 0.8% or 0.9% or 1% |
of the total mass of the tablet core.

5. The pharmaceutical formulation according to claim 3 or 4 comprising levocetirizine dihydrochloride in the amount of 5 mg and montelukast sodium in the amount equivalent to 10 mg of the montelukast free acid.

6. The pharmaceutical formulation according to any of claims 1-5 for use in treating asthma or allergic rhinitis.

7. A method for preparing the pharmaceutical formulation for oral administration according to claim 1 which comprises levocetirizine or a pharmaceutically acceptable salt thereof and montelukast or a pharmaceutically acceptable salt thereof, **characterized in that** the method comprises the steps of:
(I) mixing levocetirizine or a pharmaceutically acceptable salt thereof and at least one pharmaceutically acceptable additive,
(II) mixing montelukast or a pharmaceutically acceptable salt thereof and at least one pharmaceutically acceptable additive,
(III) blending the mixture obtained in stage (I) with the mixture obtained in stage (II) and at least one pharmaceutically acceptable additive,
(IV) tableting the mixture obtained in stage (III), and
(V) film-coating the tablets.

8. The method of claim 7 wherein the pharmaceutically acceptable additive is selected from a group comprising: microcrystalline cellulose, powdered cellulose, silicified microcrystalline cellulose, ethers of cellulose including hydroxypropyl cellulose and hydroxypropyl methylcellulose, lactose, lactose monohydrate, lactose anhydrous, spray dried lactose, croscarmellose or its salt.

9. The method of claim 7 or 8 wherein blending steps (I), (II) and (III) are followed by sieving.

10. The method of any of the claims 7-9 wherein the stages (I) and (II) are preceded by manually mixing of the ingredients.

11. The method of any of the claims 7-10 wherein the pharmaceutically acceptable salt of levocetirizine is levocetirizine dihydrochloride and the pharmaceutically acceptable salt of montelukast is montelukast sodium.

## Patentansprüche

1. Pharmazeutische Formulierung zur oralen Verabreichung in Form einer Filmtablette, erhalten durch direkte Verpressung des Tablettenkerns, enthaltend
(a) Levocetirizin oder ein pharmazeutisch akzeptables Salz davon, zusammen mit
(b) Montelukast oder einem pharmazeutisch akzeptablen Salz davon,
wobei (a) und (b) zusammen im Tablettenkern enthalten sind
und wobei der Gehalt an Montelukast-Verunreinigung C der Formel (III) 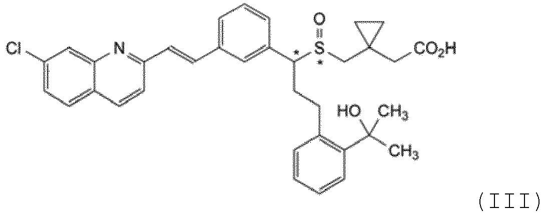 nicht mehr als 0,5% beträgt, bezogen auf den nominalen Montelukast-Gehalt, wenn die Tablette 6 Monate lang bei 40°C±2°C und 75%±5% relativer Luftfeuchtigkeit gelagert wird.

2. Pharmazeutische Formulierung gemäß Anspruch 1, wobei das pharmazeutisch akzeptable Salz von Levocetirizin Levocetirizindihydrochlorid und das pharmazeutisch akzeptable Salz von Montelukast Montelukast-Natrium ist.

3. Pharmazeutische Formulierung gemäß einem der vorangehenden Ansprüche, umfassend
| | |
|---|---|
| Levocetirizindihydrochlorid | in einer pharmazeutisch wirksamen Menge, |
| Montelukast-Natrium | in einer pharmazeutisch wirksamen Menge, |
| Lactose-Monohydrat | in einer Menge von 10% - 90%, |
| Mikrokristalline Cellulose | in einer Menge von 1% - 76%, |
| Hydroxypropylcellulose | in einer Menge von 0,5% - 4%, |
| Croscarmellose-Natrium | in einer Menge von 0,5% - 4%, |
| Magnesiumstearat | in einer Menge von 0,5% - 1% |
bezogen auf die Gesamtmasse des Tablettenkerns.

4. Pharmazeutische Formulierung gemäß einem der vorangehenden Ansprüche, umfassend
| | |
|---|---|
| Levocetirizindihydrochlorid | in einer pharmazeutisch wirksamen Menge, |
| Montelukast-Natrium | in einer pharmazeutisch wirksamen Menge, |
| Lactose-Monohydrat | in einer Menge von 10% oder 15% oder 20% oder 25% oder 30% oder 35% oder 40% oder 45% oder 50% oder 55% oder 60% oder 65% oder 70% oder 75% oder 80% oder 85% oder 90%, |
| Mikrokristalline Cellulose | in einer Menge von 1% oder 5% oder 10% oder 15% oder 20% oder 25% oder 30% 35% oder 40% oder 45% oder 50% oder 55% oder 60% oder 65% oder 70% oder 75% oder 76%, |
| Hydroxypropylcellulose | in einer Menge von 0,5% oder 1% oder 1,5% oder 2% oder 2,5% oder 3% oder 3,5% oder 4%, |
| Croscarmellose-Natrium | in einer Menge von 0,5% oder 1% oder 1,5% oder 2% oder 2,5% oder 3% oder 3,5% oder 4%, |
| Magnesiumstearat | in einer Menge von 0,5% oder 0,6% oder 0,7% oder 0,8% oder 0,9% oder 1% |
bezogen auf die Gesamtmasse des Tablettenkerns.

5. Die pharmazeutische Formulierung gemäß Anspruch 3 oder 4, umfassend Levocetirizindihydrochlorid in einer Menge von 5 mg und Montelukast-Natrium in einer Menge, die 10 mg der freien Säure von Montelukast entspricht.

6. Die pharmazeutische Formulierung gemäß einem der Ansprüche 1-5 zur Verwendung bei der Behandlung von Asthma oder allergischer Rhinitis.

7. Verfahren zur Herstellung einer pharmazeutischen Formulierung zur oralen Verabreichung gemäß Anspruch 1, die Levocetirizin oder ein pharmazeutisch akzeptables Salz davon und Montelukast oder ein pharmazeutisch akzeptables Salz davon enthält, **dadurch gekennzeichnet, dass** das Verfahren die folgenden Schritte umfasst:
(I) Vermischen von Levocetirizin oder eines pharmazeutisch akzeptablen Salzes davon mit mindestens einem pharmazeutisch akzeptablen Zusatzstoff,
(II) Vermischen von Montelukast oder eines pharmazeutisch akzeptablen Salzes davon mit mindestens einem pharmazeutisch akzeptablen Zusatzstoff,
(III) Vermischen der in Schritt (I) erhaltenen Mischung mit der in Schritt (II) erhaltenen Mischung und mindestens einem pharmazeutisch akzeptablen Zusatzstoff,
(IV) Tablettierung der in Schritt (III) erhaltenen Mischung und
(V) Beschichtung der Tabletten mit Filmüberzug.

8. Verfahren gemäß Anspruch 7, wobei der pharmazeutisch akzeptable Zusatzstoff aus einer Gruppe ausgewählt ist, die umfasst: mikrokristalline Cellulose, pulverisierte Cellulose, verkieselte mikrokristalline Cellulose, Ether von Cellulose, einschließlich Hydroxypropylcellulose und Hydroxypropylmethylcellulose, Lactose, Lactosemonohydrat, wasserfreie Lactose, sprühgetrocknete Lactose, Croscarmellose oder ihr Salz.

9. Verfahren gemäß Anspruch 7 oder 8, wobei nach den Mischungsschritten (I), (II) und (III) ein Sieben erfolgt.

10. Verfahren gemäß einem der Ansprüche 7 bis 9, wobei den Schritten (I) und (II) ein manuelles Mischen der Bestandteile vorausgeht.

11. Verfahren gemäß einem der Ansprüche 7-10, wobei das pharmazeutisch akzeptable Salz von Levocetirizin Levocetirizindihydrochlorid und das pharmazeutisch akzeptable Salz von Montelukast Montelukast-Natrium ist.

## Revendications

1. Formulation pharmaceutique pour l'administration orale sous une forme d'un comprimé pelliculé qui peut être obtenu par compression directe du noyau de comprimé et qui comprend :
a) de la lévocétirizine ou un sel pharmaceutiquement acceptable de celle-ci, mélangé(e) conjointement avec :
b) du montélukast ou un sel pharmaceutiquement acceptable de celui-ci,
où (a) et (b) sont placés ensemble dans le noyau de comprimé, et
dans laquelle la teneur en impureté C de montélukast de formule (III) : 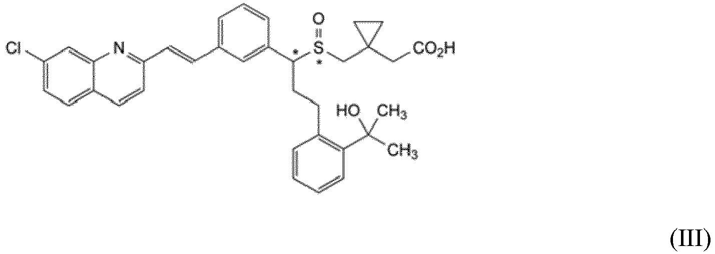 n'est pas supérieuare à 0,5% sur la base de la teneur nominale en montélukast lorsque le comprimé est stocké à 40 °C ± 2 °C et 75 % ± 5 % d'humidité relative pendant 6 mois.

2. Formulation pharmaceutique selon la revendication 1, dans laquelle le sel pharmaceutiquement acceptable de lévocétirizine est le dichlorhydrate de lévocétirizine et le sel pharmaceutiquement acceptable de montélukast est le montélukast sodique.

3. Formulation pharmaceutique selon l'une quelconque des revendications précédentes, comprenant :
| | |
|---|---|
| du dichlorhydrate de lévocétirizine | dans une quantité pharmaceutiquement efficace, |
| du montélukast sodique | dans une quantité pharmaceutiquement efficace, |
| du lactose monohydraté | dans la quantité de 10% à 90%, |
| de la cellulose microcristalline | dans la quantité de 1% à 76%, |
| de l'hydroxypropylcellulose | dans la quantité de 0,5 % à 4 %, |
| de la croscarmellose sodique | dans la quantité de 0,5 % à 4 %, |
| du stéarate de magnésium | dans la quantité de 0,5% à 1% |
de la masse totale du noyau de comprimé.

4. Formulation pharmaceutique selon l'une quelconque des revendications précédentes comprenant :
| | |
|---|---|
| du dichlorhydrate de lévocétirizine | dans une quantité pharmaceutiquement efficace, |
| du montélukast sodique | dans une quantité pharmaceutiquement efficace, |
| du lactose monohydraté | dans la quantité de 10% ou 15% ou 20% ou 25% ou 30% ou 35% ou 40% ou 45% ou 50% ou 55% ou 60% ou 65% ou 70% ou 75% ou 80% ou 85% ou 90%, |
| de la cellulose microcristalline | dans la quantité de 1% ou 5% ou 10% ou 15% ou 20% ou 25% ou 30% ou 35% ou 40% ou 45% ou 50% ou 55% ou 60% ou 65% ou 70% ou 75% ou 76%, |
| de l'hydroxypropylcellulose | dans la quantité de 0,5% ou 1% ou 1,5% ou 2% ou 2,5% ou 3% ou 3,5% ou 4%, |
| de la croscarmellose sodique | dans la quantité de 0,5% ou 1% ou 1,5% ou 2% ou 2,5% ou 3% ou 3,5% ou 4%, |
| du stéarate de magnésium | dans la quantité de 0,5 % ou 0,6 % ou 0,7 % ou 0,8 % ou 0,9 % ou 1 %, |
de la masse totale du noyau de comprimé.

5. Formulation pharmaceutique selon l'une des revendications 3 ou 4 comprenant du dichlorhydrate de lévocétirizine dans la quantité de 5 mg et du montélukast sodique dans la quantité équivalente à 10 mg du montélukast acide libre.

6. Formulation pharmaceutique selon l'une quelconque des revendications 1 à 5 pour une utilisation dans le traitement de l'asthme ou de la rhinite allergique.

7. Procédé de préparation de la formulation pharmaceutique pour l'administration orale selon la revendication 1, qui comprend de la lévocétirizine ou un sel pharmaceutiquement acceptable de celle-ci et du montélukast ou un sel pharmaceutiquement acceptable de celui-ci, **caractérisé par le fait que** le procédé comprend les étapes consistant à:
I. mélanger la lévocétirizine ou un sel pharmaceutiquement acceptable de celle-ci et au moins un additif pharmaceutiquement acceptable ;
II. mélanger le montélukast ou un sel pharmaceutiquement acceptable de celui-ci et au moins un additif pharmaceutiquement acceptable ;
III. mélanger le mélange obtenu au stade (I) avec le mélange obtenu au stade (II) et au moins un additif pharmaceutiquement acceptable ;
IV. mettre en comprimés le mélange obtenu au stade (III) ; et
V. enrober les comprimés par un film.

8. Procédé selon la revendication 7, dans lequel l'additif pharmaceutiquement acceptable est choisi dans un groupe comprenant: la cellulose microcristalline, la cellulose en poudre, la cellulose microcristalline silicifiée, les éthers de cellulose comprenant l'hydroxypropyl cellulose et l'hydroxypropyl méthyl cellulose, le lactose, le lactose monohydraté, le lactose anhydre, le lactose séché par pulvérisation, le croscarmellose ou son sel.

9. Procédé selon l'une des revendications 7 ou 8, dans lequel les étapes de mélange (I), (II) et (III) sont suivies d'un tamisage.

10. Procédé selon l'une quelconque des revendications 7 à 9, dans lequel les stades (I) et (II) sont précédées d'un mélange manuel des ingrédients.

11. Procédé selon l'une quelconque des revendications 7 à 10, dans lequel le sel pharmaceutiquement acceptable de lévocétirizine est le dichlorhydrate de lévocétirizine et le sel pharmaceutiquement acceptable du montélukast est le montélukast sodique.
